(19) Europäisches Patentamt
European Patent Office
Office européen des brevets

(11) Veröffentlichungsnummer: **0 209 000**
**B1**

(12) **EUROPÄISCHE PATENTSCHRIFT**

(45) Veröffentlichungstag der Patentschrift: **16.08.90**

(21) Anmeldenummer: **86109036.3**

(22) Anmeldetag: **02.07.86**

(51) Int. Cl.⁵: **C 07 D 215/56,**
C 07 D 401/04,
C 07 D 471/04,
C 07 D 498/06,
C 07 D 471/06,
C 07 D 455/04, C 07 C 65/11,
A 61 K 31/47, A 61 K 31/495,
A 61 K 31/435, A 61 K 31/535
// (C07D471/04, 221:00,
221:00),(C07D498/06, 265:00,
221:00),(C07D471/06, 241:00,
221:00)

(54) **Embonate von Chinoloncarbonsäuren und ihren Derivaten.**

(30) Priorität: **16.07.85 DE 3525335**

(43) Veröffentlichungstag der Anmeldung:
**21.01.87 Patentblatt 87/04**

(45) Bekanntmachung des Hinweises auf die Patenterteilung:
**16.08.90 Patentblatt 90/33**

(84) Benannte Vertragsstaaten:
**AT BE CH DE FR GB IT LI NL SE**

(56) Entgegenhaltungen:
**EP-A-0 067 666**
**FR-A-2 489 821**
**FR-A-2 500 833**
**US-A-2 397 903**
**US-A-2 641 610**

**E. Schröder et al, Pharmazeutische Chemie, Verlag G. Thieme, Stuttgart 1982, Seite 932**

(73) Patentinhaber: **BAYER AG**
**D-5090 Leverkusen 1 Bayerwerk (DE)**

(72) Erfinder: **Grohe, Klaus, Dr.**
**Am Wasserturm 10**
**D-5068 Odenthal (DE)**
Erfinder: **Voege, Herbert, Dr.**
**Martin-Buber-Strasse 41**
**D-5090 Leverkusen (DE)**
Erfinder: **Naik, Arundev Haribhai**
**Walter-Flex-Strasse 20**
**D-5090 Leverkusen (DE)**
Erfinder: **Scheer, Martin, Dr.**
**Herberts-Katernberg 7**
**D-5600 Wuppertal 1 (DE)**
Erfinder: **Federmann, Manfred, Dr.**
**Tersteegenweg 13**
**D-5600 Wuppertal 1 (DE)**

**EP 0 209 000 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Embonate von Chinoloncarbonsäuren und ihren Derivaten, Verfahren zu ihrer Herstellung sowie diese Verbindungen enthaltende orale Mittel, für die Human- und Tiermedizin.

Chinoloncarbonsäuren, ihre Derivate sowie ihre Salze sind bereits bekannt (vgl. DE—OS 30 33 157). Sie zeichnen sich durch hervorragende bakterizide Eigenschaften aus. Ihrem oralen Einsatz in Form von Lösungen, suspensionen oder Futterbeimischungen oder im Trinkwasser sind jedoch durch ihren z.T. bitteren Geschmack Grenzen gesetzt. z.B. Tieren mit hochentwickeltem Geschmackssinn wie z.B. Schweine können diese Wirkstoffe nicht ohne weiteres oral verabreicht werden.

Embonsäure und ihre Salze mit bestimmten Wirkstoffen zur verzögerten Wirkstoff-Freisetzung waren bekannt (US—P 2 397 903, Pharmazeut. Chemie (E. Schröder et al.), Verlag G. Thieme, Stuttgart 1982, S.932). Es war jedoch nicht bekannt, daß sie zur Neutralisierung des Geschmacks von Chinoloncarbonsaüren und ihren Derivaten verwendet werden können.

Es wurden die Embonate von Chinoloncarbonsäuren und ihren Derivaten der Formel (I) gefunden

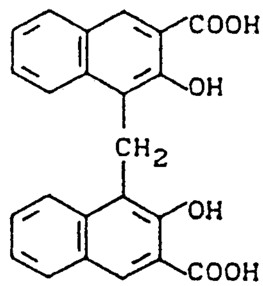

x $Y_n$ (I)

in welcher
n für 1 oder 2 steht,
Y für den Rest

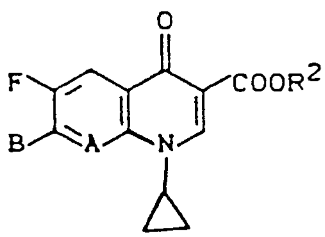

(IIa)

steht, in welcher
$R^2$ für Wasserstoff, Alkyl mit 1 bis 4 Kohlenstoffatomen
B für

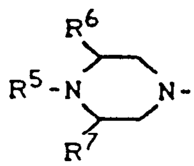

steht,
$R^5$ für Wasserstoff, Methyl oder Ethyl steht,
$R^6$ für Wasserstoff oder Methyl steht,
$R^7$ für Wasserstoff oder Methyl steht, und
A für = CH—steht.

Weiterhin wurde gefunden, daß man die Embonate von Chinoloncarbonsäuren und ihren Derivaten der Formel (I)

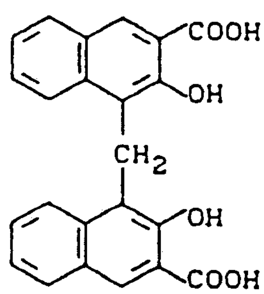

x $Y_n$ (I)

in welcher

n für 1 oder 2 steht,

Y für den oben genannten Rest der Formel IIa steht erhält, indem man Chinoloncarbonsäuren und ihre Derivate der Formel (II a) mit Embonsäure der Formel (IV)

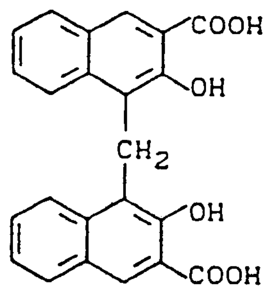

(IV)

umsetzt.

Überraschenderweise wurde gefunden, daß die neuen erfindungsgemäßen Verbindungen im Vergleich zu anderen Salzen der Chinoloncarbonsäuren geschmacksneutral sind. Sie eignen sich daher hervorragend zur Herstellung von und Verwendung in oral verabreichbaren Mitteln in der Humanmedizin und Tiermedizin. Sie eingen sich außerdem zur Herstellung von medikiertem Tierfutter oder von Präparationen die über das Trinkwasser verabreicht werden. Ferner in der Humanmedizin zur oralen Applikation dieser Derivate in Forme von Suspension. Säften, Emulsionen etc. in Fällen, wo andere galenische Zubereitungen z.B. Tabletten, Kapseln nicht angewendet werden können.

Besonders bevorzugt ist der Einsatz der erfindungsgemäßen Verbindungen in der Tiermedizin.

Dies war um so überraschender als durch Bildung anderer Salze eine Beeinflussung des Geschmacks der Chinoloncarbonsäuren oder ihrer Derivate nicht erreicht werden konnte.

Besonders genannt seinen Embonate folgender Chinoloncarbonsäuren:

1-Cyclopropyl-6-fluor-1,4-dihydro-4-oxo-7-(1-piperazinyl oder 4-Methyl- oder 4-ethyl-1-piperazinyl)-chinolin-3-carbonsäure sowie die Methyl- und Ethylester dieser Verbindungen.

Die Herstellung der Embonate von Chinoloncarbonsäuren und ihren Derivaten der Formel (I) erfolgt bevorzugt indem man Chinoloncarbonsäuren und ihre Derivate der Formeln (II a) in einem geeigneten Lösungsmittel mit Embonsäure versetzt und gegebenenfalls erhitzt.

Chinoloncarbonsäure und Embonsäure werden dabei im Verhältnis 1:1 oder 1:2 eingesetzt. Bevorzugt ist es dabei einen Überschuß von Embonsäure von 1—10%, bevorzugt 1—5% bezogen auf die Menge Embonsäure die für die obengenannten Verhältnisse notwendig ist, einzusetzen.

Ferner können die Salze von Aminochinoloncarbonsäuren mit starken Säuren wie z.B. Salzsäure, Methansulfonsäure oder Ameisensäure mit Alkali- oder Erdalkalisalzen der Embonsäure umgesetzt werden.

Als Lösungsmittel die für die Umsetzung in Frage kommen seien genannt, inerte organische Lösungsmittel wie Alkohole, z.B. Methanol, Ethanol, Ether wie Diethylether aber auch Diisopropylether, Glykolether wie Glykolmonomethylether, Kohlenwasserstoffe wie Petrolether, Toluol, Benzol, Xylol und ferner Wasser.

Die Umsetzung erfolgt bei Temperaturen von 20—150°C. Bevorzugt wird bei Siedetemperatur des Lösungsmittels gearbeitet. Es ist auch möglich die Umsetzung bei Raumtemperatur durchzuführen.

Die Wirkstoffe werden bevorzugt in Form von für Mensch und Tier geeigneten oralen Zubereitungen angewandt.

Solche Zubereitungen sind:

Orale Lösungen, Konzentrate zur oralen Verabreichung nach Verdünnung,

Emulsionen und Suspension zur oralen Anwendung.

Formulierungen bei denen der Wirkstoff in einer Salbengrundlage oder in einer Öl in Wasser oder Wasser in Öl emulsionsgrundlage verarbeitet ist;

Feste Zubereitungen wie Pulver, Premixe oder Konzentrate, Granulate, Pellets, Tabletten, Boli, Kapseln.

Orale Lösungen werden hergestellt, indem der Wirkstoff in einem geeigneten Lösungsmittel gelöst wird und eventuell Zusätze wie Lösungsvermittler, Säuren, Basen, Pufferstoffe, Antioxidantien, Konservierungsmittel zugefügt werden. Die Lösungen werden gegebenenfalls steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, lösen.

Als Lösungsvermittler seien genannt: Lösungsmittel, die die Lösung des Wirkstoffs fördern oder sein Ausfallen verhindern. Beispiele sind Polyvinylpyrrolidon, polyoxyethyliertes Rhizinusöl, polyoxyethylierte Sorbitanester.

Konservierungsmittel sind z.B. Benzylalkohol, Trichlorbutanol, p-Hydroxybenzoesäureester.

Orale Lösungen werden direkt angewendet, Konzentrate werden nach vorheriger Verdünnung auf die Anwendungskonzentration oral angewendet.

Oral anwendbare Emulsionen sind entweder vom Typ Wasser in Öl oder vom Typ Öl in Wasser.

Sie werden hergestellt, indem man den Wirkstoff in einer Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemische mit Pflanzenfettsaüren der Kettenlänge $C_{8-12}$ oder anderen speziell ausgewählten natürlichen Fettsäuren, Partialglyceridgemische gesättigter oder ungesättigter eventuell auch hdyroxylgruppenhaltiger Fettsäuren, Mono- und diglyceride der $C_{8-10}$-Fettsäuren.

Fettsäureester wie Ethylstearat, Di-n-butyl-adipat, Lauringsäurehexylester, Dipropylenglykolpelargonat, Ester einer verzweigten Fettsäure mittlerer Kettenlänge mit gesättigten $C_{16-18}$-Fettalkoholen, Isopropylmyristat, Isopropylpalmitat, Capryl/Caprinsäureester von gesättigten Fettalkoholen der Kettenlange $C_{12}$—$C_{18}$, Isopropylstearat, Ölsäureoleylester, Ölsäuredecylester, Ethyloleat, Milchsäureethylester, wachsartige Fettsäureester wie künstliches Entenbürzeldüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Olsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: Tenside (beinhaltet Emulgatoren und Netzmittel), wie

1. nichtionogene Emulgatoren, z.B. polyoxethyliertes Rizinusöl, polyoxyethyliertes Sorbitan-monooleat, Sorbitan Monostearat, Ethylalkohol, Glycerin-monostearat, Polyoxyethylstearat, Alkylphenolpolyglykolether,

2. ampholytische Emulgatoren wie Di-Na-N-lauryl-β-iminodipropionat oder Lecithin,

3. anionaktive Emulgatoren, wie Na-Laurylsulfat, Fettalkoholethersulfate, Mono/Dialkylpolyglykoletherorthophosphorsäureester-Monoethanolaminsalz,

4. kationaktive Emulgatoren wie Cetyltrimethylammoniumchlorid,

Als weitere Hilfsstoffe sind geeignet: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse;

Kolloidale Kieselsäure oder Gemische der aufgeführten Stoffe.

Oral anwendbare Suspensionen werden hergestellt, indem man den Wirkstoff in einer Trägerflüssigkeit gegebenenfalls unter Zusatz weiterer Hilfsstoffe wie Netzmittel, Farbstoffe, resorptionsfördernder Stoffe, Konservierungsstoffe, Antioxidantien, Lichtschutzmittel suspendiert.

Als Trägerflüssigkeiten seien genannt alle homogenen Lösungsmittel und Lösungsmittelgemische.

Als Netzmittel (Dispergiermittel) seien genannt:

Tenside (beinhaltet Emulgatoren und Netzmittel) wie

1. anionaktive Tenside, wie z.B. Na-Laurylsulfat, Fettalkoholethersulfate, Mono/Dialkylpolyglykoletherorthophosphorsäureester-Monoethanolaminsalz, Ligninsulfonate oder Dioctylsulfosuccinat,

2. kationaktive Tenside wie z.B. Cetyltrimethylammoniumchlorid,

3. ampholytische Tenside wie z.B. Di-Na-N-lauryl-β-iminodipropionat oder Lecithin,

4. nicht ionogene Tenside, wie z.B. polyoxethyliertes Rizinusöl, polyoxyethyliertes Sorbitan-Monoleat, Sorbitan Monostearat, Ethylalkohol, Glycerinmonostearat, Polyoxyethylenstearat, Alkylphenolpolyglykolether, Pluronic®.

Als weitere Hilfsstoffe seien die weiter oben angegebenen genannt.

Halbfeste oral verabreichbare Zubereitungen unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Innerstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmiermittel wie z.B. Magnesiumstearat, Stearinsäure, Talkum, Bentonite, zerfallsfördernde Substanzen wie Stärke oder quervernetztes Polyvinylpyrrolidon, Bindemittel

wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen. Es kann auch vorteilhaft sein, die Wirkstoffe in Formulierungen anzuwenden, die den Wirkstoff verzögert freigeben.

Die Verabreichung der Wirkstoffe erfolgt bevorzugt zusammen mit dem Futter und/oder dem Trinkwasser.

Zum Futter, zählen Einzelfuttermittel pflanzlicher Herkunft wie Heu, Rüben, Getreide,, Getreidenebenprodukte, Einzelfuttermittel tierischer Herkunft wie Fleisch, Fette, Milchprodukte, Knochenmehl, Fischprodukte, ferner die Einzelfuttermittel wie Vitamine, Proteine, Aminosäuren, z.B. DL-Methionin. Salze wie Kalk und Kochsalz. Zum Futter zählen auch Ergänzungs-, Fertig- und Mischfuttermittel. Diese enthaltend Einzelfuttermittel in einer Zusammensetzung die eine ausgewogene Erhährung hinsichtlich der Energie- und Proteinversorgung sowie der Versorgung mit Vitaminen, Mineralsalzen und Spurenelementen sicherstellen.

Die Konzentration der Wirkstoffe im Futter betägt normalerweise etwa 0,01—500 ppm, bevorzugt 0,1—50 ppm.

Die Wirkstoffe können als solche oder in Form von Prämixen oder Futterkonzentraten dem Futter zugesetzt werden.

Prämixe und Futterkonzentrate sind Mischungen des Wirkstoffes mit einem geeigneten Tragerstoff. Zu den Trägerstoffen zählen die Einzelfuttermittel oder Gemische derselben.

Sie können darüber hinaus weitere Hilfsmittel enthalten, wie z.B. — Substanzen, die die Fließfähigkeit und Mischbarkeit regulieren, wie z.b. Kieselsäuren, Bentonite, Ligninsulfonate. Darüber hinaus können Antioxydantien wie BHT oder Konservierungsmittel wie Sorbinsäure oder Calciumpropionat hinzugefügt sein.

Konzentrate zur Applikation über das Trinkwasser müssen so formuliert sein, daß heim Vermischen mit dem Trinkwasser eine klare Lösung oder eine homogene Suspension entsteht.

Als Trägerstoffe sind daher wasserslösliche Substanzen (Futterzusatzmittel) wie Zucker oder Salze (z.B. Citrate, Phosphate, Kochsalz, Na-Karbonat) geeignet.

Sie können ebenfalls Antioxydantien und Konservierungsmittel enthalten.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Wirkstoffe wirken gegen human- und tierpathogene Mikroorganismen.

Zu diesen Mikroorganismen zählen:

1. Spirochaetaceae (z.B. Treponema, Leptospira, Borrelia)
2. Spirillaceae
3. Micrococcaceae (z.B. Staphylococcen Biotyp A—F, St. hyicus)
4. Streptococcaceae (zB. Streptococcus uberis, Str. equi, Str. agalactiae, Str. dysgalactiae, Streptococcen der Lancefield Typen A—N)
5. Pseudomonaceae (z.B.Pseudomonas malei, Ps, cepacia., Ps. aeruginosa, Ps. maltophilia), Brucella wie Brucella abort, B. melitensis, B. suis, Bordetella wie Bordetella bronchiseptica, Moraxella, Acinetobacter)
6. Enterobacteriaceae (z.B. Salmonella der Typen B—E, Shigella, E. coli, Klebsiella, Proteus, Citrobacter, Edwardsiella, Haemophilus, Providencia, Yersina)
7. Vibrionaceae (z.B. Bribrio wie Vibrio chloerae), Pasteurella wie Pasteurella multocida, Aeromonas, Actinobacillus, Streptobacillus
8. Bacteroidaceae (z.B. Bacteroides, Fusobacterium),
9. Erysiphylothix, Listeria wie Listeria monocytojenes
10 Bacillaceae (z.B. Bacillus, Clostridium Typen A—D, wie Clostridium perfringens), Lactobacillaceae sowie anaerobe Coccen wie z.B. Peptostreptococcen und Peptococcen
11. Coryneforme Bakterien (z.B. Corynebacterium pyogenes)
12. Mycobacteriaceae (z.B. Mycobacterium bovis, M. avium, M. tuberculosis)
13. Actinomyceae (z.B. Actinomyces bovis, A. israelii)
14. Nocardiaceae (z.B. Norcardia facinica, N. asteroides)
15. Rickettsiaceae (z.B. Coxiella, Rickettsia)
16. Bartonellaceae (z.B. Bartonella)
17. Chlamydiaceae (z.B. Chlamydia psittaci)
18. Mycoplasmataceae (z.B. Mycoplasma mycoides, M. agalactiae, M. gallisepticum).

Human- und tierpathogenen Mikroorganismen Können als Mono- oder Mischinfektionen Krankheitsbilder bei folgenden Organsystemen der Tiere hervorrufen:

Lunge und Trachialraum, Verdauungssystem wie Magen und Darm, Brust und Euter, Genitalsystem wie Uterus, Weichteile wie Haut, Muskeln, Nägel, Krallen, Hufe, aktiver und passiver Bewegungsapparat wie Knochen, Muskeln, Sehnen, Gelenke, Urogenitalsystem wie Niere, Harnleiter, Harnröhre, Nervensystem, Gehörapparat, Augen.

Wie bereits erwähnt werden die Wirkstoffe zur Bekämpfung bakterieller Erkrankungen bei Mensch und Tier verwendet. Zu den Tieren zählen:

Säugetiere wie z.B. Rinder, Pferde, Schweine, Schafe, Ziergen, Hunde, Katzen, Kamele, Pelztiere wie Nerz Chinchilla, Zootiere und Labortiere wie z.B. Mäuse und Ratten;

Vögel, wie z.B. Gänse, Hühner, Truthühner, Enten, Tauben, Zoovögel, Laborvögel wie z.B. Papageien und Wellensittiche;

Fische, wie z.B. z.B. Karpfen, Forellen, Lachse, Schleien, Aale, ferner Zierfische und Aquarienfische;

Reptilien wie z.B. Krokodile, Schlangen.

Zu den bakteriellen Erkrankungen bei Tieren zählen z.B. Schweinedysenterie: Geflügelspirochätose; Leptospirose bei Rind, Schwein, Pferd, Hund: Campylobacter-Enteritis beim Rind; Campylobacter-Abort bei Schaf und Schwein; Campylobacter- Hepatitis der Hühner; Infektionen der Haut; Pyodermien beim Hund; Otitis externa; Mastitis des Rindes, der Schafe und der Ziege; Streptokokkenmastitis; Streptokokkeninfektionen des Pferdes, beim Schwein und anderen Tierarten; Pneumokokkeninfektion des Kalbs, und bei anderen Tierarten; Malleus; Konjunktivitis; Enteritiden; Pneumonien; Brucellose bei Rind, Schaf, Schwein; Rhinitis atrophicans des Schweins; Salmonellose bei Rind, Pferd, Schaf, Huhn und anderen Tierarten; Septikämien; Escherichia coli Infektion beim Ferkel; Metritis-Mastitis-Agalaktic-(MMA)-Syndrom; Klebsiella Infektionen; Pseudotuberkulose; Infektiöse Pleuropneumonie; Primäre Pasteurellosen; Fohlenlähme; Nekrobazillose beim Rind und bei Haustieren; Leptospirose; Rotlauf des Schweins und anderer Tierarten, Listeriose; Milzbrand; Clostridiosen; Tetanusinfektionen; Botulismus; Infektionen mit Corynebacterium pyogenes; Tuberkulose bei Rind, Schwein, Geflügel und anderen Tierarten; Paratuberkulose der Wiederkäuer; Nokardiose; Q-Fieber; Ornithose-Psittakose; Enzephalomycelitis; Mykoplasmose des Rindes und anderer Tiere, Enzootische Pneumonie der Schweine.

Beispiel 1

Salz A: (n in Formel (I) steht für 1)

Eine suspension von 62,1 g (0,173 Mol) 1-Cyclopropyl-6-fluor-1,4-dihydro-4-oxo-7-(4-ethyl-1-piperazinyl)-chinolin-3-carbonsäure und 67,3 g (0,173 Mol) Embonsäure (Pamoic acid, 4,4'-Methylen-bis-(3-hydroxy)-2-naphthoesäure, Fluka Nr. 45150) in 800 ml Glykolmonomethylether wird 30 Minuten refluxiert. Der Nierderschlag wird kalt abgesaugt, mit Ethanol gewaschen und im Vakuum bei 120°C bis zur Gewichtskonstanz getrocknet. Es werden 127,6 g (98,6% der Theorie) Salz A vom Zersetzungspunkt 232—235°C erhalten.

Beispiel 2

Salz B: (n in Formel (I) steht für 2)

Eine Suspension von 71,8 g (0,2 Mol) 1-Cyclopropyl-6-fluor-1,4-dihdyro-4-oxo-7-(4-ethyl-1-piperaz inyl)-chinolin-3-carbonsäure (*1*) und 40 g (0,103 Mol) Embonsäure (*2*) in 500 ml Glykolmonomethylether wird 1 Stunde refluxiert. Der Niederschlag wird kalt abgesaugt, mit Ethanol gewaschen und im Vakuum bei 120°C bis zur Gewichtskonstanz getrocknet. Es werden 107,2 g (96,9% der Theorie) Salze B vom Zersetzungspunkt 226—228°C erhalten.

$C_{61}H_{60}F_2N_6O_{12}$ (1106)

Ber.: C, 66,16; H, 5,42; N, 7,59; F, 3,43

Gef.: C, 66,0; H, 5,6; N, 7,5; F, 3,4;

Analog der obengenannten Vorschriften wurden folgende Salze von 1-Cyclopropyl-6-fluor-1,4-dihydro-4-oxo-7-(4-ethyl-1-piperazinyl)-chinolincarbonsäure hergestellt: Salze mit den Basen (NaOH; KOH; $Ca(OH)_2$:

Salze mit den Säuren (HCl; HBr; $HNO_3$; $CH_3SO_3H$; $CH_3COOH$). All diese Salze zeigten bei einer durchgeführten Geschmacksprobe deutliche bitteren Geschmack.

Die Salze der Beispiele 1 und 2 zeigten demgegenüber keinen Geschmack.

**Patentansprüche**

1. Embonate von Chinoloncarbonsäuren und ihren Derivaten der Formel (I)

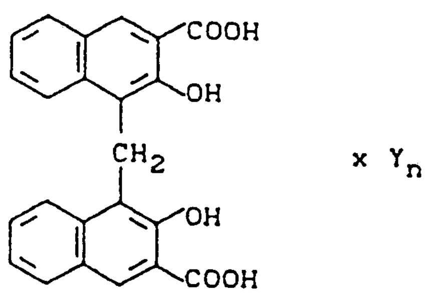

(I)

in welcher
n für 1 oder 2 steht,
Y für den Rest der Formel

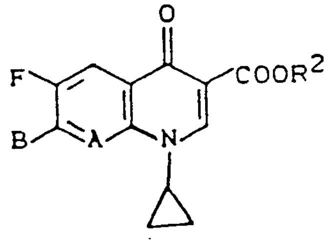

(IIa)

steht, in welcher
$R^2$ für Wasserstoff, Alkyl mit 1 bis 4 Kohlenstoffatomen steht
B für

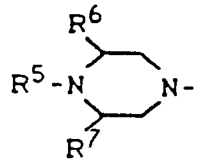

steht,
$R^5$ für Wasserstoff, Methyl oder Ethyl steht,
$R^6$ für Wasserstoff oder Methyl steht,
$R^7$ für Wasserstoff oder Methyl steht, und
A für = CH— steht.

2. Verfahren zur Herstellung von Embonaten von Chinoloncarbonsäuren und inhren Derivaten der Formel (I)

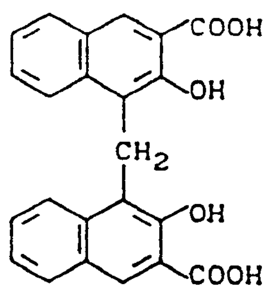

$\times Y_n$ (I)

in welcher
n für 1 oder 2 steht,
Y für den Rest

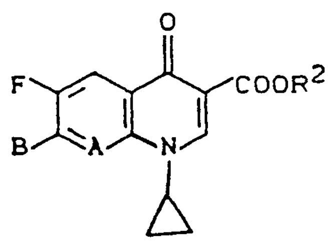

(IIa)

steht, in welcher
$R^2$ für Wasserstoff, Alkyl mit 1 bis 4 Kohlenstoffatomen steht,
B für

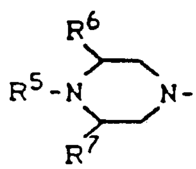

steht,

$R^5$ für Wasserstoff, Methyl oder Ethyl steht,
$R^6$ für Wasserstoff oder Methyl steht,
$R^7$ für Wasserstoff oder Methyl steht, und
A für = CH— steht.

dadurch gekennzeichnet, daß man Chinoloncarbonsäuren und ihre Derivate der Formeln (II a) mit Embonsäure der Formel (IV)

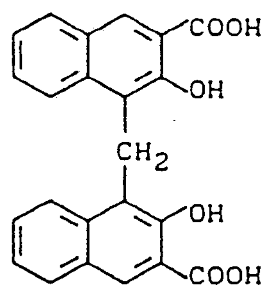

(IV)

umsetzt.

3. Orale bakterizide Zubereitungen zur Anwendung auf dem Gebiet der Medizin und Veterinärmedizin, die Embonate von Chinoloncarbonsäuren und ihren Derivaten der Formel (I) gemäß Anspruch 1 enthalten.

4. Zubereitungen gemäß Anspruch 3, dadurch gekennziechnet, daß sie Embonate von 1-Cyclopropyl-6-fluor-1,4-dihydro-4-oxo-7-(1-piperazinyl) oder (1-[4-ethyl-piperazinyl])-chinolin-3-carbonsäure enthalten.

5. Antibakterielle Arzneimittel, Futtermittel oder Trinkwässer, gekennzeichnet durch einen Gehalt an mindestens einer Zubereitung gemäß Anspruch 3.

6. Verwendung der Zubereitungen gemäß Anspruch 3 in oralen Darreichungsformen sowie in Konzentraten zur Herstellung derselben.

7. Verfahren zur Herstellung von Zubereitungen gemäß Anspruch 3, dadurch gekennzeichnet, daß man Embonate von Chinoloncarbonsäuren der Formel I gemäß Anspruch 1 mit Streck- und/oder Verdünnungsmitteln vermischt.

**Revendications**

1. Embonates d'acides quinolone-carboxyliques et leurs dérivés de formule (I)

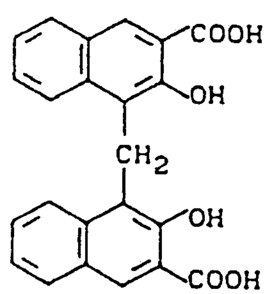

x $Y_n$

(I)

dans laquelle
n signifie 1 ou 2,
Y représente le reste de formule

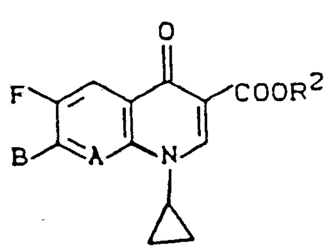

(IIa)

dans lequel

$R^2$ représente l'hydrogène, un groupe alkyle avec 1 à 4 atomes de carbone

B représente

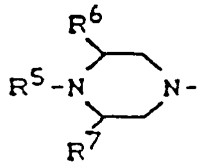

$R^5$ représente l'hydrogène, un groupe méthyle ou éthyle,

$R^6$ représente l'hydrogène ou un groupe méthyle,

$R^7$ représente l'hydrogène ou un groupe méthyle, et

A représente =CH—.

2. Procédé pour la fabrication des embonates d'acides quinolone-carboxyliques et leurs dérivés de formule (I)

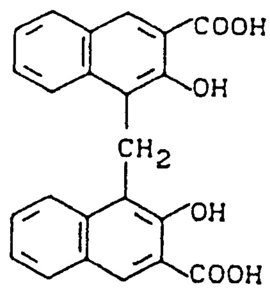

x $Y_n$ (I)

dans laquelle

n signifie 1 ou 2,

Y représente le reste

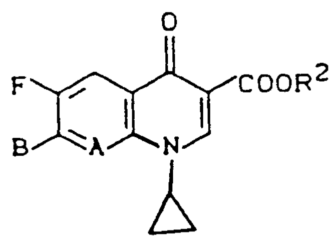

(IIa)

dans lequel

$R^2$ représente l'hydrogène, un groupe alkyle avec 1 à 4 atomes de carbone

B représente

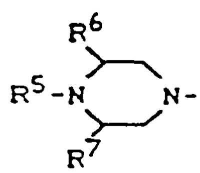

$R^5$ représente l'hydrogène, un groupe méthyle ou éthyle,

$R^6$ représente l'hydrogène ou un groupe méthyle,

$R^7$ représente l'hydrogène ou un groupe méthyle, et

A représente =CH—, caractérisé en ce que l'on fait réagir les acides quinolone-carboxyliques et leurs dérivés de formule (IIa) avec l'acide embonique de formule (IV)

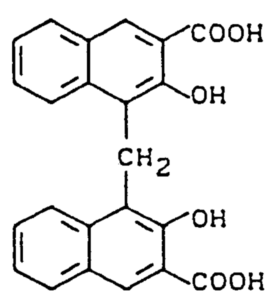

(IV)

3. Préparations bactéricides pour administration orale applicables dans le domaine de la médecine humaine et de la médicine vétérinaire, contenant les embonates d'acides quinolone-carboxyliques et leurs dérivés de formule (I) selon la revendication 1.

4. Préparations selon la revendication 3, caractérisée en ce qu'elles contiennent des embonates d'acide 1-cyclopropyl-6-fluoro-1,4-dihydro-4-oxo-7-(1-pipérazinyl) ou (1-[4-éthyl-pipérazinyl])-quinoléine-3-carboxylique.

5. Médicaments, aliments ou eau de boisson antibactériens, caractérisés par une teneur en au moins une préparation selon la revendication 3.

6. Utilisation des préparations selon la revendication 3 dans des formes pour administration orale ainsi que dans des concentrés pour la fabrication de celles-ci.

7. Procédé pour la fabrication de préparations selon la revendication 3, caractérisé en ce que l'on mélange les embonates d'acide quinolonecarboxyliques de formule (I) selon la revendication 1 avec des agents d'allongement ou de dilution.

## Claims

1. Embonates of quinolonecarboxylic acids and of their derivatives of the formula (I)

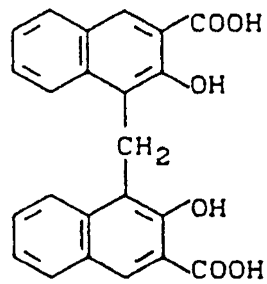

x $Y_n$ (I)

in which

n represents 1 or 2,

Y represents the radical of the formula

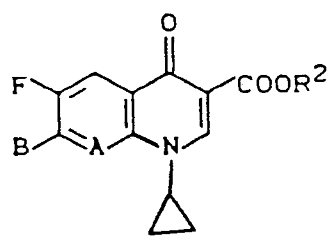

(IIa)

in which

$R^2$ represents hydrogen, alkyl having 1 to 4 carbon atoms,

B represents

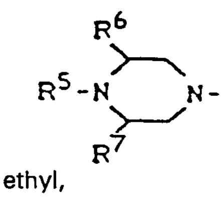

$R^5$ represents hydrogen, methyl or ethyl,

$R^6$ represents hydrogen or methyl,

$R^7$ represents hydrogen or methyl, and

A represents =CH—.

2. Process for the preparation of embonates of quinolonecarboxylic acids and of their derivatives of the formula (I)

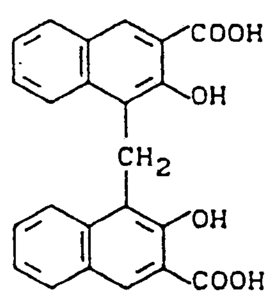

(I)

in which
n represents 1 or 2,
Y represents the radical

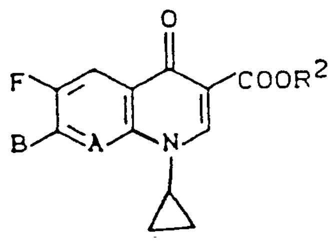

(IIa)

in which
$R^2$ represents hydrogen, alkyl having 1 to 4 carbon atoms,
B represents

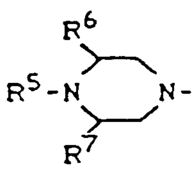

$R^5$ represents hydrogen, methyl or ethyl,
$R^6$ represents hydrogen or methyl,
$R^7$ represents hydrogen or methyl, and
A represents =CH—, characterized in that quinolonecarboxylic acids and their derivatives of the formulae (II) are reacted with embonic acid of the formula (IV)

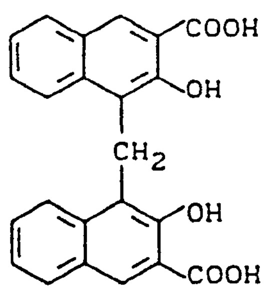

(IV)

3. Oral bactericidal formulations for use in the area of medicine and veterinary medicine, which contain embonates of quinolonecarboxylic acids and of their derivatives of the formula (I) according to Claim 1.

4. Formulations according to Claim 3, characterized in that they contain embonates of 1-cyclopropyl-6-fluoro-1,4-dihydro-4-oxo-7-(1-piperazinyl) or (1-[4-ethyl-piperazinyl])-quinoline-3-carboxylic acid.

5. Antibacterial medicaments, feedstuffs or drinking water, characterized in that they contain at least one formulation according to Claim 3.

6. Use of the formulations according to Claim 3 in oral administration forms and in concentrates for the preparation thereof.

7. Process for the preparation of formulations according to Claim 3, characterized in that embonates of quinolonecarboxylic acids of the formula I according to Claim 1 are mixed with extenders and/or diluents.